# EUROPÄISCHE PATENTANMELDUNG

(11) **EP 2 770 043 A1**
(43) Veröffentlichungstag der Anmeldung: **27.08.2014**
(21) Anmeldenummer: 13156112.8
(22) Anmeldetag: 21.02.2013
(51) Int. Cl.: C11D 3/382, C11D 3/384, C11D 7/44, C11D 7/46, A61K 8/97, A61K 8/98

(54) **Detergens**

(71) Anmelder: LINA LINE ® E.U., 1220 Wien (AT)
(72) Erfinder: Kavalar, Wolfgang, 9500 Villach (AT); Kurzmayr, Herbert Friedrich, 1220 Wien (AT)
(74) Vertreter: Sonn & Partner Patentanwälte

(57) **Zusammenfassung**

Beschrieben wird ein Detergens, insbesondere Waschmittel, Reinigungsmittel oder Kosmetikum, enthaltend
- Waschnuss-Tensid und
- Molke und/oder Permeat.

## Beschreibung

Die vorliegende Erfindung betrifft Detergenzien, insbesondere Waschmittel, Reinigungsmittel oder Kosmetika, auf Basis von Waschnuss-Tensiden.

Fast alle gängigen Wasch- oder Reinigungsmittel, aber auch viele Kosmetika, enthalten Enzyme, Tenside, Konservierungsmittel und Duftstoffe, die Ursache für eine Allergie sein können. Eine Waschmittelallergie ist eine Überreaktion des Immunsystems auf derartige Tenside, Duftstoffe, Seifenbestandteile oder Aufheller. Diese Substanzen sind Kontaktallergene, die eine Überempfindlichkeitsreaktion auslösen. Symptome einer Waschmittelallergie können leichte oder starke Hautrötungen, Juckreiz oder sogar eine Neurodermitis sein.

Abhilfe können hier die Verwendung von Detergenzien, insbesondere in Waschmitteln, Reinigungsmitteln und Kosmetika schaffen, die auf einer rein pflanzlichen Basis beruhen. Zusätzlich werden oft die Verwendung eines sogenannten Ökowaschballs oder einer Waschnuss oder Waschnussextrakten als ein komplett natürliches Waschmittel empfohlen. Menschen mit einer Waschmittelallergie sollten außerdem auf Weichspüler verzichten. Gerade bei Menschen mit sensibler Haut führen die Rückstände des Weichspülers in der Kleidung zu Hautirritationen und Juckreiz. In der Regel werden aber die in Waschmitteln enthaltenen Enzyme als die Hauptverursacher von Waschmittelallergien angesehen. Diese Enzyme werden vom Immunsystem als fremd erkannt ("Allergene") und lösen eben diese Überempfindlichkeitsreaktion aus. Auch die synthetischen Tenside in Waschmitteln, die dazu dienen, die Löslichkeit von Schmutz- und Fettpartikeln zu erhöhen, können Grund für Juckreiz und Hautveränderungen sein.

Da heute die Waschmaschinen auf Energiesparkurs kaum Wasser zum Waschen zur Verfügung stellen, die Wäschefasern im geöffneten Zustand die Waschmittelreste aufnehmen und nicht mehr ausgespült werden, können dadurch Allergien, Juckreiz und Hautveränderungen auftreten.

Zur Vermeidung derartiger Waschmittelallergien oder anderer Hautkrankheiten, die auf Detergenzien, insbesondere in Waschmitteln, Reinigungsmitteln und Kosmetika, zurückgeführt werden, wurden zunehmend besonders hautverträgliche Mittel für die sensible Haut auf den Markt gebracht. Waschnüsse oder Waschmittel auf Basis von Waschnuss-Extrakten werden als besonders geeignet für Allergiker angesehen. Hierzu wurden sogar spezielle Behälter für Waschnüsse vorgeschlagen, die in herkömmlichen Wachmaschinen verwendbar sind (EP 2 042 640 A1).

Es ist Aufgabe der vorliegenden Erfindung, ein Detergens, insbesondere als Waschmittel, Reinigungsmittel oder Kosmetikum, auf Waschnussbasis mit verbesserten Eigenschaften zur Verfügung zu stellen, bei denen die Vorteile derartiger Produkte, enthaltend dieses Detergens (keine Allergieauslöser, Umwelt- und Hautfreundlichkeit, etc.) beibehalten werden, jedoch die Wasch- und Reinigungskraft noch weiter verbessert werden.

Demgemäß betrifft die Erfindung ein Detergens, insbesondere Waschmittel, Reinigungsmittel oder Kosmetikum" enthaltend
- Waschnuss-Tensid und
- Molke und/oder Permeat.

Das Detergens, insbesondere Waschmittel, Reinigungsmittel oder Kosmetikum, gemäß der vorliegenden Erfindung stellt eine Weiterentwicklung der auf Waschnüssen bzw. Waschnuss-Tensiden basierenden Mitteln dar, welches alle Vorteile der Waschnuss-Mittel aufweist, jedoch eine überraschende und deutliche Verbesserung in der Wasch- bzw. Reinigungswirkung zeigt.

Das Detergens, insbesondere Waschmittel, Reinigungsmittel oder Kosmetikum, gemäß der vorliegenden Erfindung kann in allen Formen und Gebinden angeboten werden, die für Produkte, enthaltend Detergenzien, also für die Reinigung und Waschen verwendet werden, üblich sind. Die wesentlichen Produkt-Kategorien der Detergens-Produkte gemäß der vorliegenden Erfindung sind dabei Waschmittel, Reinigungsmittel und (insbesondere für Wasch- und Reinigungszwecke zu verwendende) Kosmetika. Ein "Waschmittel" ist dabei generell ein Mittel, welches zum Waschen und Reinigen verwendet wird, vorwiegend von Textilien. Ganz allgemein werden unter Waschmitteln Gemische verschiedener Substanzen in flüssiger, gelartiger oder pulverförmiger Art verstanden, die zum Reinigen von Textilien verwendet werden. Sie enthalten waschaktive Substanzen, insbesondere Tenside, welche in der Lage sind, Verunreinigungen von Textilien zu lösen. Waschmittel und Reinigungsmittel sind "Detergenzien" im Sinne der Verordnung (EG) Nr. 648/2004 des Europäischen Parlaments und des Rates vom 31. März 2004 über Detergenzien ("Detergenzien-VO") angesehen werden. Darin wird ein "Detergens" als Stoff oder Zubereitung angesehen, welcher/welche Seifen und/oder andere Tenside enthält und für Wasch- und Reinigungsprozesse bestimmt ist. Detergenzien (Waschmittel), insbesondere auch das Waschmittel, Reinigungsmittel oder Kosmetikum gemäß der vorliegenden Erfindung, können, wie erwähnt, unterschiedliche Formen haben (Flüssigkeit, Pulver, Paste, Riegel, Tafel, geformte Stücke, Figuren usw.) und für Haushaltszwecke, zu kosmetischen Zwecken oder für institutionelle oder industrielle Zwecke vertrieben oder verwendet werden. Im Rahmen der vorliegenden Erfindung sind unter Detergenzien alle zu derartigen Wasch- und Reinigungsprozessen vorgesehenen Mittel (gemäß Detergenzien-VO), jedoch auch Kosmetika und andere Produkte zu verstehen, die das erfindungsgemäße Detergens als wesentlichen Inhaltsstoff enthalten (also zB zu einem Anteil, der über 10%, vorzugsweise über 20 %, insbesondere über 30 % (bzw. sogar über 50%), liegt).

Die in Detergenzien, insbesondere in Waschmitteln, Reinigungsmitteln oder Kosmetika, enthaltenen "Tenside" sind dabei gemäß Detergenzien-VO die "in Detergenzien verwendeten organischen Stoffe und/oder Zubereitungen mit grenzflächenaktiven Eigenschaften, die aus einer oder mehreren hydrophilen und einer oder mehreren hydrophoben Gruppen solcher Art und Größe bestehen, dass sie die Fähigkeit besitzen, die Oberflächenspannung von Wasser zu verringern, monomolekulare Streuungs- oder Adsorptionsschichten an der Wasser/Luft-Grenzfläche zu bilden, Emulsionen und/oder Mikroemulsionen und/oder Micellen zu bilden und sich an Wasser/Festkörper-Grenzflächen anzulagern".

Die Detergenzien, insbesondere Waschmittel, Reinigungsmittel und Kosmetika, gemäß der vorliegenden Erfindung enthalten Waschnuss-Tenside als Tenside, d.h. aus Waschnüssen extrahierte Saponine, und entsprechen der Detergenzien-VO bzw. vorzugsweise auch anderen, nationalen Gesetzen, deren Einhaltung für die Zulässigkeit des Verkaufes von Detergenzien, insbesondere Waschmittel, Reinigungsmittel und Kosmetika, eingehalten werden müssen (z.B. dem deutschen Wasch- und Reinigungsmittelgesetz).

Da beim Waschen mit der Waschnuss und deren Produkte die Haut,- Haar - und Wäsche-Faser geschlossen bleibt, können sich keine Rückstände in der Wäsche, im Haar oder der Haut festsetzen. Das erklärt, weshalb die Waschnüsse so Haut- und Faser-schonend sind. Die Reinigungskraft des erfindungsgemäßen Detergens ist gegenüber Waschnüssen alleine durch die Kombination mit Molke um ein Vielfaches verstärkt; jedoch ist das erfindungsgemäße Detergens dennoch sehr Haut-, Haar-, Faser- und Umwelt-schonend.

Das erfindungsgemäße Detergens kann, wie erwähnte, für alle Arten von Waschmittel verwendet werden, insbesondere für Colorwaschmittel, Vollwaschmittel, Waschpulver, Waschmittel für Feinwäsche, Wolle, Seide, Schwarzwäsche, dunkle Wäsche, helle Wäsche, etc. Bevorzugte Reinigungsmittel mit dem erfindungsgemäßen Detergens Universalreiniger (Allzweckreiniger), Glas-Fenster- Waschanlagen- Küchen-Badreiniger, Handgeschirrspülmittel, Geschirrspültabs, Geschirrspülpulver, Maschinenreiniger, Melkanlagenreiniger, Reiniger für alle NIRO-Geräte und Gefäße, Töpfe, WC-Reiniger, Auto-Reiniger, etc. Bevorzugte Kosmetika sind Körperpflegemittel, insbesondere alle Arten von Seifen, Shampoos, Duschbäder, Schaumbäder, Reinigungsmilch und Bade-bzw. Peelingsalzen, etc., einschließlich Shampoos bzw. Pflegemittel für Haus- und Nutztiere, insbesondere Hundeshampoos.

Die im erfindungsgemäßen Detergens, insbesondere Waschmittel, Reinigungsmittel oder Kosmetikum, verwendeten Waschnuss-Tenside werden aus den Früchten des Waschnussbaumes, den Waschnüssen, gewonnen.

Der Waschnussbaum (Sapindus mukorossi) sowie weitere Spezien gehören zur Art Waschnussbaum (Gattung: Seifenbaum (Sapindus); Familie Seifenbaumgewächse (Sapindaceae); Ordnung: Seifenbaumartige (Sapindales)) und ist ein Baum der tropischen und subtropischen Regionen Asiens. Die Früchte der Pflanze werden, wie erwähnt, als "Waschnüsse" oder "Seifennüsse" bezeichnet und werden seit Jahrhunderten zum Waschen, aber auch für medizinische Zwecke, verwendet. Waschnussbäume können bis zu 25 Meter hoch werden, einen Stammumfang von 3 bis 5 Metern erreichen und dabei siebzig Jahre und älter werden. Die ersten Waschnüsse trägt der Baum in der Regel nach zehn Jahren. Die Waschnüsse sind orangefarbene, klebrige Nüsse, die etwa haselnussgroß sind und im September geerntet werden. Nach dem Trocknen sind die Früchte nicht mehr klebrig und rotbraun bis dunkelbraun.

Die Schale der Nüsse enthält, je nach Art bis zu 16/17 % (S. mukorossi) Saponine (auch abhängig von der Erntezeit und dem Alter des Baums).

Die vorliegende Erfindung beruht darauf, die Waschnuss-Tenside mit Molke bzw. Permeat zu kombinieren. Dabei ist die Verwendung von Molke klar bevorzugt, da die Waschwirkung von Molke gegenüber der Verwendung von Permeat nochmals deutlich verbessert ist. Die Verwendung von Permeat bringt erfindungsgemäß jedoch auch schon einen bedeutenden Vorteil gegenüber herkömmlichen Waschnuss-Tensiden. Überraschender Weise bietet die Kombination der Waschnuss-Tenside mit anderen Molkeähnlichen Produkten wie z.B. Buttermilch oder Magermilch keine Vorteile bzw. eine deutlich verschlechterte Waschwirkung oder Handhabbarkeit.

Molke ist die wässrige grünlich-gelbe Restflüssigkeit, die bei der Käseherstellung entsteht. Sie ist der flüssige Teil, der nach der Gerinnung der Milch zu Käse oder Quark abgesondert werden kann. Es gibt zwei Sorten von Molke: die Süßmolke (auch Labmolke), die entsteht, wenn man Milch mit Lab zur Käseherstellung dicklegt, und die Sauermolke, die entsteht, wenn man Milch mit Milchsäurebakterien behandelt. Obgleich erfindungsgemäß die Süßmolke bevorzugt ist, kann auch die Sauermolke effizient im vorliegenden Detergens verwendet werden. Molke kann erfindungsgemäß sowohl in flüssiger Form als auch in Form von Molkepulver verwendet werden.

Permeat wird hauptsächlich durch Mikrofiltration im Rahmen der Milchverarbeitung gewonnen. Als Trennkriterium dient dabei die Partikelgröße, zB von 0,1, bis 0,3 µm. Bei der Permeat-Herstellung wird die Magermilch in zwei Ströme, Permeat und Retentat, geteilt. Das Permeat ist die Milch, die die Membran passiert und zu 99,5 Prozent frei von Bakterien und Sporen ist. Das Permeat besteht zum größten Teil aus Wasser, enthält 3,3 bis 4,5 % Milchzucker, 4, 9-5, 2 % Salze und Asche (bei einem pH-Wert von etwa 6,67). Im Retentat verbleiben hauptsächlich Caseine und kleinere Anteile an Molkenprotein sowie Lactose, im Permeat befinden sich hauptsächlich Mineralsalze, Lactose, Molkenproteine und kleine Anteile an Caseinsubmizellen.

Der Zusatz von Molke/Permeat wird mengenmäßig so vorgenommen, dass die Kombination die für die jeweilige Aufgabe erwünschte Waschleistung aufweist.

Gemäß einer bevorzugten Ausführungsform enthält das erfindungsgemäße Detergens, insbesondere Waschmittel, Reinigungsmittel oder Kosmetikum, 5 - 50 Gew.-% Molke, bevorzugter 10 - 40 Gew.-% Molke, noch bevorzugter 20 - 35 Gew.-% Molke, insbesondere 20 - 30 Gew.-% Molke.

Das erfindungsgemäße Detergens, insbesondere Waschmittel, Reinigungsmittel oder Kosmetikum, weist vorzugsweise einen Saponin-Gehalt von 5 - 70 %, vorzugsweise 10 - 50 %, insbesondere von 15 - 30 %, auf. Der Saponin-Gehalt wird erfindungsgemäß als %-Gehalt der im Detergens, insbesondere Waschmittel, Reinigungsmittel oder Kosmetikum, enthaltenen Seifenstoffe, gemessen als °Öchsle, bestimmt. Im einfachsten Fall kann man den Saponin-Gehalt daher folgendermaßen ermitteln: Da aus der Waschnuss ein Zuckertensid entsteht, kann man den Zuckergrad mit einem Refraktometer messen und dabei einen Öchsle-Wert erhalten. Der dabei ermittelte Öchsle-Wert minus 10 % ergibt dann den Saponin-Gehalt. Dabei gelten Detergenzien, insbesondere Waschmittel, Reinigungsmittel oder Kosmetika, mit 15 bis 30 % als Konzentrate; mit über 30 % Saponinen als Hochkonzentrate.

Das erfindungsgemäße Detergens, insbesondere Waschmittel, Reinigungsmittel oder Kosmetikum, kann, wie erwähnt, in allen gängigen Verkaufs-, Transport- oder Handelsformen für Detergenzien, insbesondere Waschmittel, Reinigungsmittel oder Kosmetika, bereitgestellt werden. Demgemäß wird das erfindungsgemäße Detergens, insbesondere Waschmittel, Reinigungsmittel oder Kosmetikum, vorzugsweise als Verkaufsprodukt in einem Behältnis abgepackt, das ein Volumen von 100 ml bis 10 1, vorzugsweise 250 ml bis 5 1, insbesondere 500 ml bis 2 1, aufweist. Dies sind die typischen Volumina, in denen Detergenzien, insbesondere Waschmittel, Reinigungsmittel oder Kosmetika, gleich ob Trocken- oder Flüssigmittel, zum Verkauf für den Endkunden im Geschäft angeboten werden.

Nach der Produktion kann das erfindungsgemäße Detergens, insbesondere Waschmittel, Reinigungsmittel oder Kosmetikum, zu Transportzwecken in größere Industriegebinde abgefüllt werden. Hierfür sind vor allem Intermediate Bulk Container (IBC) besonders geeignet. IBC werden für Transport und Lagerung flüssiger und rieselfähiger Stoffe verwendet. Sie werden üblicherweise bei der Produktion und zum Transport von Chemikalien, Lebensmitteln, Kosmetik und Pharmazeutika eingesetzt. Je nach Bauweise und Ausführung haben die IBC ein Volumen von 500 bis zu 3000 Litern. Ferner sind IBC für den Transport von Gefahrgut zugelassen. Hierzu ist es allerdings nötig, alle zweieinhalb bzw. fünf Jahre eine Wiederholungsprüfung (WHP) durchzuführen. Hauptvorteil von IBC gegenüber den zylindrischen Fässern ist die bessere Ausnutzung des Füllraumes durch die kubische Ausführung. Vier Stahlfässer mit jeweils 200 Litern Volumen auf eine Palette gestellt benötigen genauso viel Platz wie ein IBC mit 1000 Litern. Dem Entleerer wird die Arbeit erleichtert, da er für 1000 Liter Produkt nur einen Arbeitsgang benötigt anstelle von fünf Arbeitsgängen bei Fässern für die gleiche Menge. Die meisten IBC haben darüber hinaus den Vorteil, dass diese nach Benutzung zu reinigen sind und somit mehrfach verwendet werden können. Das Bewegen erfolgt mit Gabelstaplern oder Hubwagen und die IBC sind bauartbedingt stapelbar. IBC sind z.B. als Kombinations-IBC (Palette mit Kunststofftank und einem einfachen Gitterkäfig oder Rohrrahmen), Kunststoff-IBC (mit freitragender Blase), metallene IBC (in der Regel aus Edelstahl), beheizbare IBC, Flexible IBC (aus gewebten und vernähten Polypropylen-Werkstoff) oder faltbare IBC (faltbarer Kunststoffbehälter und sterilisierter Kunststoffbeutel (Inliner) mit aseptischem Ventil) erhältlich.

Vorzugsweise wird das erfindungsgemäße Detergens, insbesondere Waschmittel, Reinigungsmittel oder Kosmetikum, entweder als Flüssig-Detergens, insbesondere Flüssig-Waschmittel, Flüssig-Reinigungsmittel und Flüssig-Kosmetikum, oder als Detergens-Pulver, insbesondere Waschmittel-, Reinigungsmittel- oder Kosmetikum- Pulver, zur Verfügung gestellt.

Das Detergens, insbesondere Waschmittel, Reinigungsmittel oder Kosmetikum, gemäß der vorliegenden Erfindung ist vor allem auch durch seine biologische Natur und reiz- und allergenarme Eigenschaften gekennzeichnet. Es ist praktisch in der Anwendung, einfach in der Herstellung und umweltschonend, sowohl in der Herstellung als auch bei und vor allem nach dem Waschprozess. Das erfindungsgemäße Produkt ist ein natürliches Produkt mit hoher Qualität und Wasch- bzw Reinigungskraft. Diese Produkte sind für mindestens 2 Jahre in geschlossenen Zustand (6 Monate nach Öffnen) garantiert haltbar.

Beim Waschprozess können die Faserstrukturen der zu reinigenden Textilien in der Regel geschlossen erhalten bleiben, wodurch keine potenziell allergenen Stoffe in den Fasern haften bleiben. Ergebnis des Waschprozesses ist eine weiche Wäsche, wodurch die Behandlung mit einem Weichspüler entfallen kann und ein leichteres Bügeln ermöglicht wird.

Das erfindungsgemäße Detergens, insbesondere Waschmittel, Reinigungsmittel oder Kosmetikum, kann somit seine Wirkung entfalten, ohne dass es die ansonsten erforderlichen weiteren Inhaltsstoffe üblicher Detergenzien enthalten muss. Insbesondere können einige oder sogar alle diese Zusatzstoffe im erfindungsgemäßen Waschmittel weggelassen werden. Vorzugsweise ist das erfindungsgemäße Detergens, insbesondere Waschmittel, Reinigungsmittel oder Kosmetikum, daher frei von
- Bleichstoffen und/oder
- synthetischen Tensiden und/oder
- Farbstoffen, insbesondere synthetischen Farbstoffen, und/oder
- allergieauslösenden Substanzen (gemäß ISO 9235) und/oder
- Duftstoffen, insbesondere synthetischen Duftstoffen und/oder
- reizenden und ätzenden Stoffen, insbesondere Chlor.

Besonders bevorzugt sind erfindungsgemäße Detergenzien, insbesondere Waschmittel, Reinigungsmittel oder Kosmetika,, worin zwei, drei, viel oder alle fünf der erwähnten Stoffklassen (Bleichstoffe, synthetische Tenside, Farbstoffe, allergieauslösende Substanzen und Duftstoffe) fehlen bzw. nicht hinzugefügt werden. Derartige Detergenzien, insbesondere Waschmittel, Reinigungsmittel oder Kosmetika, können für Kinderwäsche oder für den Apothekerbedarf dienen.

Vorzugsweise werden dem erfindungsgemäßen Detergens, insbesondere Waschmittel, Reinigungsmittel oder Kosmetikum, natürliche Duftstoffe zugesetzt, insbesondere Kamille-, Lavendel-, wilde Rose-, Apfelblüte-, Pfirsichblüte-, Grüner Tee-, Lemongrass-, Lime-, Flieder-Duftstoffe oder Mischungen davon, wobei die Duftstoffe vorzugsweise zu 0,01 bis 1 Gew.-%, insbesondere zu 0,05 bis 0,5 Gew.-%, im Detergens, insbesondere Waschmittel, Reinigungsmittel oder Kosmetikum, enthalten sind. Vorzugsweise sind die Duftstoffe Öko-Düfte gemäß ISO 9235. Die Duftrichtungen können selbstverständlich jederzeit verändert werden, stammen aber vorzugsweise immer aus der Gruppe der Öko-Düfte.

Gemäß einer besonderen Ausführungsform enthält das erfindungsgemäße Detergens, insbesondere Waschmittel, Reinigungsmittel oder Kosmetikum, Ca-Sorbat und/oder Na-Benzoat, vorzugsweise jeweils von 0,01 bis 1 Gew.-%, insbesondere zu 0,05 bis 0,5 Gew.-%.

Gemäß einer weiteren bevorzugten Ausführungsform wird das erfindungsgemäße Detergens, insbesondere Waschmittel, Reinigungsmittel oder Kosmetikum, als Flüssig-Detergens, insbesondere Flüssig-Waschmittel, Flüssig-Reinigungsmittel oder Flüssig-Kosmetikum, mit einem sauren pH vorgesehen, vorzugsweise mit einem pH von 3 bis 7, noch bevorzugter von 4 bis 6, insbesondere von 4 bis 6.

Gemäß einem weiteren Aspekt betrifft die vorliegende Erfindung ein Zweikomponenten-Detergensset, umfassend
- eine Waschnuss-Komponente und
- eine Molke- oder Permeat- Komponente.

Mit dem erfindungsgemäßen Set kann das Detergens, insbesondere Waschmittel, Reinigungsmittel oder Kosmetikum, unmittelbar vor dem Wasch- bzw. Reinigungsprozess individuell zusammengestellt werden, insbesondere in Anbetracht dem Verschmutzungsgrad der Wäsche. Die Komponenten können als Flüssigkeiten oder als Pulver vermischt und dem Waschprozess zugeführt werden bzw. erst im Zuge des Waschprozesses miteinander in Kontakt kommen.

Vorzugsweise liegen die Komponenten des Detergenssets in fester Form vor, insbesondere als Trockenpulver.

Obgleich das erfindungsgemäße Set sowohl Waschnüsse selbst (oder Waschnussschalen) als auch Waschnuss-Tenside als Waschnuss-Komponente enthalten kann, ist das Vorsehen von Waschnuss-Tensiden als Waschnusskomponente klar bevorzugt.

Gemäß einem weiteren Aspekt betrifft die Erfindung ein Verfahren zur Reinigung von Textilien, wobei die Textilien in Kontakt mit Wasser, Molke oder Permeat und Waschnuss-Tensid (bzw. dem erfindungsgemäßen Detergens) gebracht und solange bewegt werden, bis die Textilien aufgrund der Reinigungswirkung der Kombination von Molke oder Permeat mit Waschnuss-Tensiden gereinigt sind. Die "Bewegung" findet dabei natürlich üblicher Weise in Waschmaschinen statt, jedoch ist das erfindungsgemäße Detergens bzw. Detergensset auch ausgezeichnet zur Handwäsche geeignet, insbesondere aufgrund seiner natürlichen Eigenschaften. Die Dosierung ist dabei abhängig z.B. von der Menge und vom Verschmutzungsgrad der Wäsche, der Wasserhärte, dem Waschnuss-Tensid-Gehalt des Detergens und von der vorgesehenen Waschtemperatur. Beispielsweise können - bei der Ausführungsform Flüssig-Detergens - 10 bis 100 ml, vorzugsweise 20 bis 80 ml, insbesondere 30 bis 70 ml, des erfindungsgemäßen Detergens 5 kg einer durchschnittlich verschmutzten Wäsche in die Einspülkammer einer Waschmaschine zugegeben werden. Die Waschdauer wird prinzipiell analog zu der Waschdauer herkömmlicher Detergenzien eingestellt, jedoch vorzugsweise eine etwas längere Waschdauer als bei herkömmlichen Detergenzien gewählt.

Schließlich betrifft die vorliegende Erfindung auch ein Verfahren zur Herstellung eines Detergens, insbesondere Waschmittel, Reinigungsmittel oder Kosmetikum, gemäß der vorliegenden Erfindung, wobei Waschnüsse mit Wasser oder Molke oder Permeat extrahiert werden, wobei ein Extrakt mit Waschnuss-Tensiden erhalten wird, wobei der Extrakt gegebenenfalls anschließend getrocknet wird, und danach gegebenenfalls mit Molke oder Permeat gemischt wird. Vorzugsweise wird dann die Mischung erhitzt, beispielsweise auf zumindest 60°C, insbesondere auf zumindest 80°C, dann gefiltert, beispielsweise durch Sackfilter, Schichtenfilter, Druckfilter, Kerzenfilter, Kieselgutfilter oder Rotationsfilter, und abgefüllt.

Die Erfindung wird anhand der folgenden Beispiele und der Zeichnungsfiguren, auf die sie selbstverständlich nicht beschränkt ist, näher erläutert.

Es zeigen:
Fig. 1 zeigt die gemischte und erhitzte Waschmittel-Mischung (A), die Mischung bei der Filterung (B), den Filter (C) und das erhaltene Endprodukt (D);
Fig. 2 zeigt die gemischte und erhitzte Waschmittel-Mischung (A), die Mischung nach der Filterung (B), den Filter (C) und das erhaltene Endprodukt (D);
Fig. 3 zeigt die gemischte und erhitzte Waschmittel-Mischung (A), die Mischung bei der Filterung (B), die Mischung nach der Filterung (C) und das erhaltene Endprodukt (D);
Fig. 4 zeigt die gemischte und erhitzte Waschmittel-Mischung (A), die Mischung bei der Filterung (B), den Filter (C) und das erhaltene Endprodukt (D);

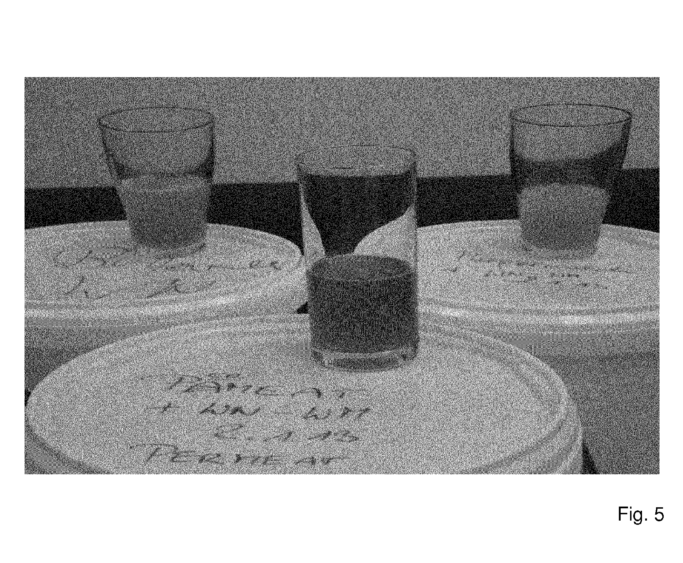

Fig. 5 zeigt die Waschmittel gemäß Beispiel 2 (vorne), 3 (hinten links) und 4 (hinten rechts);
Fig. 6 zeigt eine Probewäsche mit dem Molke-Waschmittel gemäß Beispiel 1;

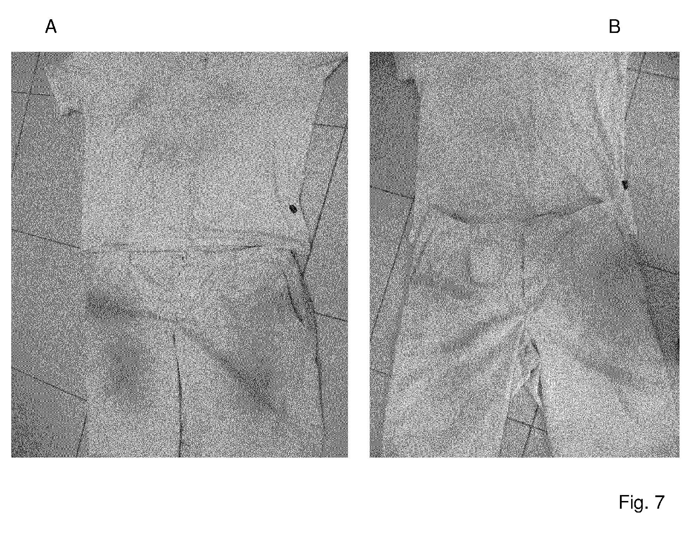

Fig. 7 zeigt eine Probewäsche mit dem Permeat-Waschmittel gemäß Beispiel 2.

### Beispiele

### 1. Waschnuss-Molke-Waschmittel

In diesem Beispiel wurde ein erfindungsgemäßes Flüssigwaschmittel hergestellt, das aus 75% Waschnuss-Tensid und 25 % Molke besteht.

Das Waschmittel wurde zubereitet, indem 25 kg Molke zu Waschnuss-Tensid zugesetzt wurde, wobei 100 kg Flüssig-Waschmittel erhalten wurden. Dann wurde das Mittel auf über 60° erhitzt, gefiltert und abgefüllt.

Als das Waschnuss-Tensid mit Molke in Verbindung kam, trübte es nur ganz wenig ein, entwickelte sich wie beim Waschnuss-Tensid ohne Molke-Zusatz, jedoch mit etwas mehr Schaumbildung.

| | |
|---|---|
| Waschnuss-Tensid: | 41 % Saponin-Gehalt |
| Molke: | 39 % Zucker-Gehalt |
| Waschnuss-Molke-Waschmittel: | 39 % Saponin-Gehalt |
| pH-Wert: | 3,92 |

Das Waschmittel war trotz Filtrierung leicht eingetrübt und zeigte die folgenden Eigenschaften bzw. Waschperformance:
Geruch in der nassen Wäsche: Säuerlich, bitter
Geruch in der trockenen Wäsche: Neutral
Waschergebnis: starke Fett- und Schmutz-lösende Waschkraft
Wäsche - weich: kuschelweiche Wäsche

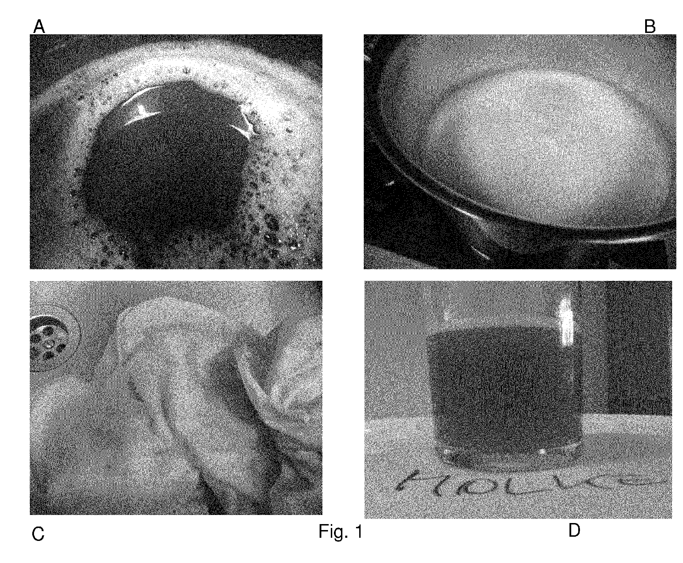

In Fig. 1 ist die Herstellung auch fotografisch dargestellt: Das Produkt zeigte leichte Eintrübung, jedoch gute Produzierbarkeit, sehr gute Filtrierbarkeit und einfache Handhabung.

### 2. Waschnuss-Permeat-Waschmittel

In diesem Beispiel wurde ein erfindungsgemäßes Flüssigwaschmittel hergestellt, das aus 75% Waschnuss-Tensid und 25 % Permeat besteht.

Das Waschmittel wurde zubereitet, indem 25 kg Permeat zu Waschnuss-Tensid zugesetzt wurde, wobei 100 kg Flüssig-Waschmittel erhalten wurden. Dann wurde das Mittel auf über 60° erhitzt, gefiltert und abgefüllt.

Als das Waschnuss-Tensid mit Permeat in Verbindung kam, trübte es nur ganz wenig ein, entwickelte sich wie beim Waschnuss-Tensid ohne Permeat-Zusatz. Beim Erhitzen auf über 60° ergab sich keine Änderung, das Waschmittel wurde nur etwas klarer.

| | |
|---|---|
| Waschnuss-Tensid: | 41 % Saponin-Gehalt |
| Permeat: | 20 % Zucker-Gehalt |
| pH: | 5,8 |
| Waschnuss-Permeat-Waschmittel: | 39 % Saponin-Gehalt |
| pH-Wert: | 4,14 |

Das Waschmittel war trotz Filtrierung leicht eingetrübt und zeigte die folgenden Eigenschaften bzw. Waschperformance:
Geruch in der nassen Wäsche: Säuerlich, bitter
Geruch in der trockenen Wäsche: Neutral
Waschergebnis: gute Waschkraft, jedoch schlechter als in Beispiel 1.
Wäsche - weich: weich, jedoch nicht so weich wie in Beispiel 1.

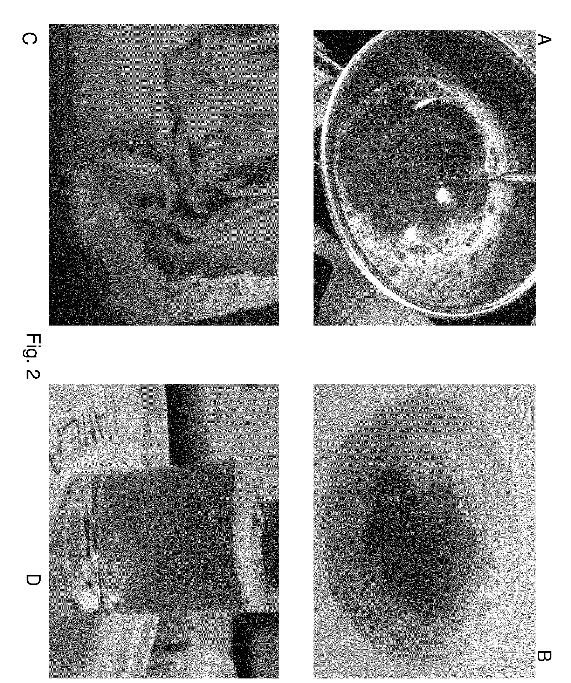

In Fig. 2 ist die Herstellung auch fotografisch dargestellt: Das Produkt zeigte leichte Eintrübung, jedoch gute Produzierbarkeit, sehr gute Filtrierbarkeit und einfache Handhabung.

### 3. Waschnuss-Buttermilch-Waschmittel

In diesem Beispiel wurde ein erfindungsgemäßes Flüssigwaschmittel hergestellt, das aus 75 % Waschnuss-Tensid und 25 % Buttermilch besteht.

Das Waschmittel wurde zubereitet, indem 25 kg Buttermilch zu Waschnuss-Tensid zugesetzt wurde, wobei 100 kg Flüssig-Waschmittel erhalten wurden. Dann wurde das Mittel auf über 60° erhitzt, gefiltert und abgefüllt.

Als das Waschnuss-Tensid mit Buttermilch in Verbindung kam, flockte es aus, wurde sauer und fiel aus. Beim Erhitzen auf 85° löste sich die Buttermilch, ein Teil wurde bröckelig ("Käse"). Das erhaltene Waschmittel wurde sehr trübe, die Durchführung der Filtrierung erwies sich als sehr schwierig.

| | |
|---|---|
| Waschnuss-Tensid: | 41 % Saponin-Gehalt |
| Waschnuss-Buttermilch-Waschmitte1: | 40 % Saponin Gehalt |
| pH-Wert: | 4,28 |

Das Waschmittel war trotz Filtrierung trübe und flockig; es befanden sich kleinere Flocken im Waschmittel.

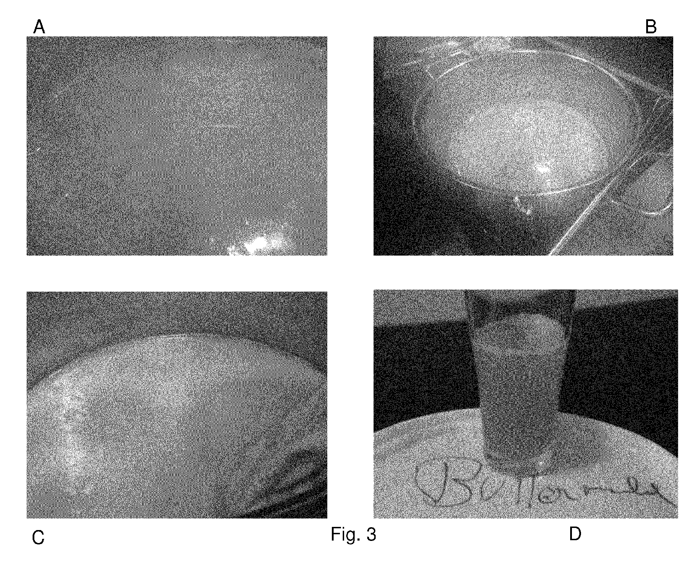

In Fig. 3 ist die Herstellung auch fotografisch dargestellt.

### 4. Waschnuss-Magermilch-Waschmittel

In diesem Beispiel wurde ein erfindungsgemäßes Flüssigwaschmittel hergestellt, das aus 75 % Waschnuss-Tensid und 25 % Magermilch besteht.

Das Waschmittel wurde zubereitet, indem 25 kg Magermilch zu Waschnuss-Tensid zugesetzt wurde, wobei 100 kg Flüssig-Waschmittel erhalten wurden. Dann wurde das Mittel auf über 60° erhitzt, gefiltert und abgefüllt.

Als das Waschnuss-Tensid mit Magermilch in Verbindung kam, stockte es und flockte es aus, wurde sauer und fiel aus. Beim Erhitzen auf 85° löste sich die Magermilch, ein Teil wurde bröckelig ("Käse"). Das erhaltene Waschmittel wurde sehr trübe, die Durchführung der Filtrierung erwies sich als sehr schwierig.

| | |
|---|---|
| Waschnuss-Tensid: | 41 % Saponin-Gehalt |
| Waschnuss-Magermilch-Waschmittel: | 40 % Saponin Gehalt |
| pH-Wert: | 4,58 |

Das Waschmittel war trotz Filtrierung trübe und flockig; es befanden sich größere Flocken im Waschmittel.

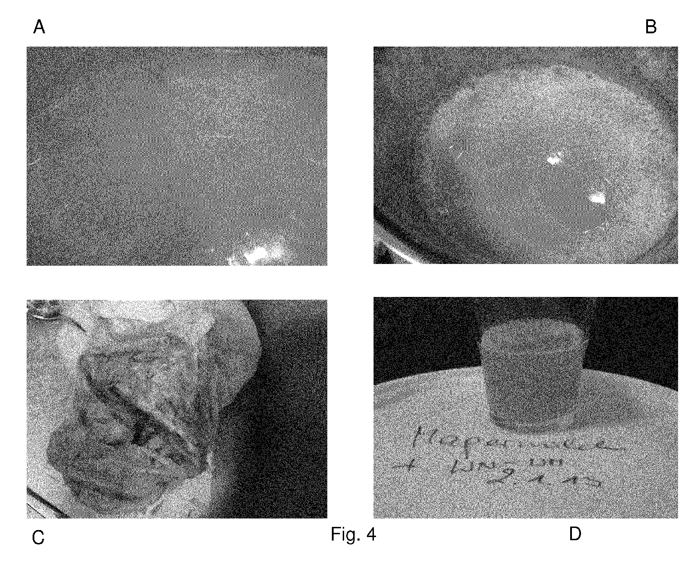

In Fig. 4 ist die Herstellung auch fotografisch dargestellt.

### 5. Probewäsche mit Waschnuss-Molke und Waschnuss-Permeat-Waschmittel

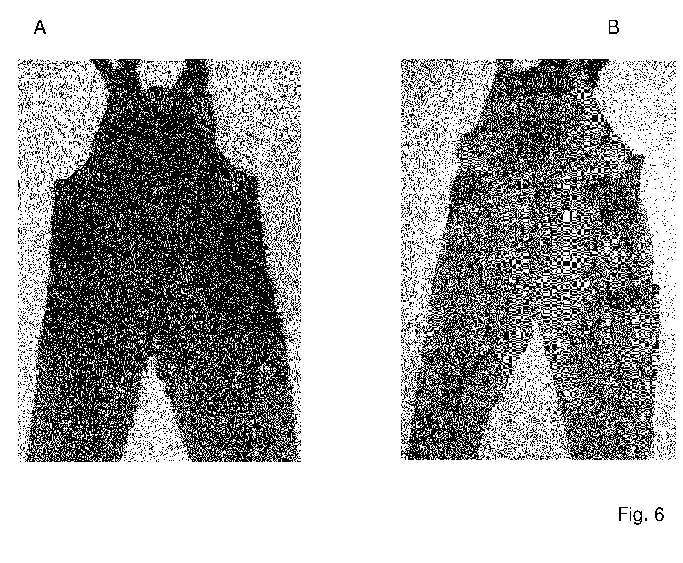

In diesem Beispiel wurden die Waschmittel gemäß Beispiel 1 und 2 unterzogen. Stark verschmutzte Wäsche wurde jeweils bei 90°C für 90 min gewaschen. Das Ergebnis ist in den Figuren 6 (Molke-Waschmittel) und 7 (Permeat-Waschmittel) dargestellt (A: vorher; B: nach der Wäsche).

### 6. Waschnuss-Molke-Allzweck-Reiniger

In diesem Beispiel wurde ein erfindungsgemäßer Allzweck-Reiniger hergestellt, der 20-40 % erfindungsgemäßes Detergens (Waschnusstensid/Molke 75:25 gemäß Beispiel 1 hergestellt), 0,2 % Ca-Sorbat, 0,1 % Na-Benzoat Zuckertensid, und 0,2 % ÖKO Duft gemäß ISO 9235 enthält.

Der Allzweck-Reiniger wurde z.B. anhand von Fliesenböden getestet und zeigte eine starke Reinigungswirkung, wobei sich herausstellte, dass dieser biologische Allzweckreiniger keine Fliesenfugen löst, jedoch bei Schmutz und Kalkrückständen sehr gute Reinigungswirkung aufweist.

### 7. Waschnuss-Molke-Kosmetikum (Shampoo)

Ein Waschnuss-Molke-Kosmetikum wurde als Shampoo für trockenes und normales Haar hergestellt, indem das erfindungsgemäße Waschnuss-Molke-Detergens (75:25 gemäß Beispiel 1 hergestellt) mit Zuckertensid, Zitronensäure, Jojobaöl, Xanthan, Konjac-Wurzel, Sesamöl und einer Duftkomposition aus Öko-Düften gemäß ISO 9235 vermischt wurde.

Die Benutzung des Kosmetikums durch Testpersonen ergab nach der Haarwäsche weiches und seidenglänzendes Haar, wobei das Shampoo auch gegen Schuppen wirkte. Bei dieser Anwendung zeigte sich vor allem der Umstand als vorteilhaft, dass die Haarstruktur geschlossen bleibt und durch die Kombination aus Waschnuss-Tensid und Molke ein zusätzlicher pflegender Effekt eintritt.

## Patentansprüche

1. Detergens, insbesondere Waschmittel, Reinigungsmittel oder Kosmetikum, enthaltend
- Waschnuss-Tensid und
- Molke und/oder Permeat.

2. Detergens, insbesondere Waschmittel, Reinigungsmittel oder Kosmetikum, nach Anspruch 1, **dadurch gekennzeichnet, dass** es Molke enthält, vorzugsweise 5 - 50 Gew.-% Molke, noch bevorzugter 10 - 40 Gew.-% Molke, insbesondere 20 - 30 Gew.-% Molke.

3. Detergens, insbesondere Waschmittel, Reinigungsmittel oder Kosmetikum, nach Anspruch 1 oder 2, **dadurch gekennzeichnet, dass** es einen Saponin-Gehalt von 5 - 70 %, vorzugsweise 10 - 50 %, insbesondere von 15 - 30 %, aufweist, wobei der Saponin-Gehalt als %-Gehalt der im Detergens, insbesondere Waschmittel, Reinigungsmittel oder Kosmetikum, enthaltenen Seifenstoffe, gemessen als °Öchsle, bestimmt wird.

4. Detergens, insbesondere Waschmittel, Reinigungsmittel oder Kosmetikum, nach einem der Ansprüche 1 bis 3, **dadurch gekennzeichnet, dass** es in einem Behältnis abgepackt ist, das ein Volumen von 100 ml bis 10 1, vorzugsweise 250 ml bis 51, insbesondere 500 ml bis 2 1, aufweist.

5. Detergens, insbesondere Waschmittel, Reinigungsmittel oder Kosmetikum, nach einem der Ansprüche 1 bis 4, **dadurch gekennzeichnet, dass** es ein Flüssig-Detergens, insbesondere Waschmittel, Reinigungsmittel oder Kosmetikum, ist.

6. Detergens, insbesondere Waschmittel, Reinigungsmittel oder Kosmetikum, nach einem der Ansprüche 1 bis 4, **dadurch gekennzeichnet, dass** es ein Waschmittelpulver ist.

7. Detergens, insbesondere Waschmittel, Reinigungsmittel oder Kosmetikum, nach einem der Ansprüche 1 bis 6, **dadurch gekennzeichnet, dass** es frei ist von
- Bleichstoffen und/oder
- synthetischen Tensiden und/oder
- Farbstoffen, insbesondere synthetischen Farbstoffen, und/oder
- allergieauslösenden Substanzen und/oder
- Duftstoffen, insbesondere synthetischen Duftstoffen und/oder
- reizenden und ätzenden Stoffen, insbesondere Chlor.

8. Detergens, insbesondere Waschmittel, Reinigungsmittel oder Kosmetikum, nach einem der Ansprüche 1 bis 7, **dadurch gekennzeichnet, dass** es natürliche Duftstoffe enthält, insbesondere Kamille-, Lavendel-, wilde Rose-, Apfelblüte-, Pfirsichblüte-, Grüner Tee-, Lemongrass-, Lime-, Flieder-Duftstoffe oder Mischungen davon, wobei die Duftstoffe vorzugsweise zu 0,01 bis 1 Gew.-%, insbesondere zu 0,05 bis 0,5 Gew.-%, im Detergens, insbesondere Waschmittel, Reinigungsmittel oder Kosmetikum, enthalten sind.

9. Detergens, insbesondere Waschmittel, Reinigungsmittel oder Kosmetikum, nach einem der Ansprüche 1 bis 8, **dadurch gekennzeichnet, dass** es Ca-Sorbat und/oder Na-Benzoat enthält, vorzugsweise jeweils von 0,01 bis 1 Gew.-%, insbesondere zu 0,05 bis 0,5 Gew.-%.

10. Detergens, insbesondere Waschmittel, Reinigungsmittel oder Kosmetikum, nach einem der Ansprüche 1 bis 9, **dadurch gekennzeichnet, dass** es als Flüssig-Detergens, insbesondere Flüssig-Waschmittel, Flüssig-Reinigungsmittel oder Flüssig-Kosmetikum, vorliegt und einen sauren pH aufweist, vorzugsweise einen pH von 3 bis 7, noch bevorzugter von 4 bis 6, insbesondere von 4 bis 6.

11. Zweikomponenten-Detergensset, umfassend
- eine Waschnuss-Komponente und
- eine Molke- oder Permeat- Komponente.

12. Detergensset nach Anspruch 11, **dadurch gekennzeichnet, dass** die Komponenten in fester Form, insbesondere als Trockenpulver, vorliegen.

13. Detergensset nach Anspruch 11 oder 12, **dadurch gekennzeichnet, dass** die Waschnusskomponente ein Waschnuss-Tensid ist.

14. Verfahren zur Reinigung von Textilien, **dadurch gekennzeichnet, dass** die Textilien in Kontakt mit Wasser, Molke oder Permeat und Waschnuss-Tensid gebracht und solange bewegt werden, bis die Textilien aufgrund der Reinigungswirkung der Kombination von Molke oder Permeat mit Waschnuss-Tensiden gereinigt sind.

15. Verfahren zur Herstellung eines Detergens, insbesondere Waschmittel, Reinigungsmittel oder Kosmetikum, nach einem der Ansprüche 1 bis 10, **dadurch gekennzeichnet, dass** Waschnüsse mit Wasser oder Molke oder Permeat extrahiert werden, wobei ein Extrakt mit Waschnuss-Tensiden erhalten wird, wobei der Extrakt gegebenenfalls anschließend getrocknet wird, und danach gegebenenfalls mit Molke oder Permeat gemischt wird.
